# EUROPEAN PATENT SPECIFICATION

(11) **EP 3 704 088 B1**
(45) Date of publication and mention of the grant of the patent: **16.07.2025**
(21) Application number: 17930200.5
(22) Date of filing: 03.11.2017
(51) Int. Cl.: C07C 69/708, C07C 69/76, A01N 25/02

(54) **SOLVENTS FOR AGRICULTURAL APPLICATIONS AND PESTICIDE FORMULATIONS**
LÖSUNGSMITTEL FÜR LANDWIRTSCHAFTLICHE ANWENDUNGEN UND PESTIZIDFORMULIERUNGEN
SOLVANTS POUR APPLICATIONS AGRICOLES ET FORMULATIONS PESTICIDES

(43) Date of publication of application: 09.09.2020
(73) Proprietor: Dow Global Technologies LLC, Midland, MI 48674 (US)
(72) Inventor: LU, Wei, Shanghai 201203 (CN); REN, Hua, Shanghai 201203 (CN); ZHONG, Ling, Shanghai 201203 (CN); MU, Jianhai, Shanghai 201203 (CN); YUN, Dong, Shanghai 201203 (CN)
(74) Representative: Boult Wade Tennant LLP
(86) International application number: PCT/CN2017/109246
(87) International publication number: WO 2019/084895

(56) References cited:
- CN-A- 1 693 322
- CN-A- 101 855 200
- CN-A- 102 007 450
- JP-A- 2009 161 679
- US-A- 2 504 151
- US-A1- 2003 109 415
- KOZUKI Y. ET AL.: "A predictive solubility tool for pesticide emulsifiable concentrate Formulations", JOURNAL OFASTM INTERNATIONAL, vol. 6, no. 9, 31 August 2009 (2009-08-31), pages 69 - 81, XP009159561

## Description

### Field

The present invention relates to pesticide formulations.

### Introduction

Aromatic solvents, such as xylene, have been regularly utilized in agricultural applications. Xylene is commonly used as it provides several advantages, such as dissolving a variety of active ingredients and being easily emulsified in water on dilution. However, there are number of disadvantages to xylene's use as a solvent such as flammability, combustibility, and toxicity. Thus, there is a need for solvent alternatives to xylene for use in agricultural applications.

KOZUKI Y. et al. ; "A predictive solubility tool for pesticide emulsifiable concentrate Formulations", Journal of ASTM International, vol. 6, no. 9, 31 August 2009, pages 69-81 describes a predictive solubility tool.

A variety of alternatives to xylene have been considered such as aromatic fluids produced from petroleum-based raw materials such as naphtha solvents composed of C₉-C₁₃ aromatics. However, such solvents still have issues such as low flash point and high toxicity.

It would be desirable to have new solvents for agricultural applications having good dissolution properties while also having an improved environmental/health/safety profile.

### Summary

Described herein are solvents for pesticides that, in some embodiments, have a dissolving ability comparable to that of xylene while also being more environmentally friendly.

Described herein isa solvent for pesticides that comprises an ether ester of Formula 1: 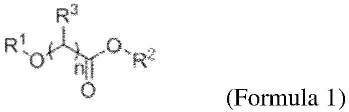 wherein R¹ and R² are each independently one of an alkyl group having 1 to 4 carbon atoms or an aryl group, wherein R³ is hydrogen, methyl, or ethyl, and wherein n is 1 to 6. In some embodiments, R¹ and R² are ethyl groups, R³ is hydrogen, and n is 2.

In the first aspect, the present invention provides a pesticide formulationas defined in claim 1 of the claims appended hereto.

These and other embodiments are described in more detail in the Detailed Description.

### Detailed Description

As used herein, "a," "an," "the," "at least one," and "one or more" are used interchangeably. Thus, for example, an aqueous composition that includes particles of "a" hydrophobic polymer can be interpreted to mean that the composition includes particles of "one or more" hydrophobic polymers.

The numerical ranges disclosed herein include all values from, and including, the lower and upper value. For ranges containing explicit values (e.g., 1 to 7), any subrange between any two explicit values is included (e.g., 1 to 2; 2 to 6; 5 to 7; 3 to 7; 5 to 6; etc.).

The terms "comprising," "including," "having," and their derivatives, are not intended to exclude the presence of any additional component, step or procedure, whether or not the same is specifically disclosed. In order to avoid any doubt, all compositions claimed through use of the term "comprising" may include any additional additive, adjuvant, or compound, whether polymeric or otherwise, unless stated to the contrary. In contrast, the term, "consisting essentially of" excludes from the scope of any succeeding recitation any other component, step, or procedure, excepting those that are not essential to operability. The term "consisting of" excludes any component, step, or procedure not specifically delineated or listed. The term "or," unless stated otherwise, refers to the listed members individually as well as in any combination. Use of the singular includes use of the plural and vice versa.

Unless stated to the contrary, implicit from the context, or customary in the art, all parts and percents are based on weight and all test methods are current as of the filing date of this disclosure.

"Solvent" and like terms mean a substance that is capable of dissolving another substance (i.e., a solute) to form an essentially uniformly dispersed mixture (i.e., solution) at the molecular or ionic size level.

Described herein are solvents for pesticides. In some embodiments, a solvent for pesticides comprises an ether ester of Formula 1: 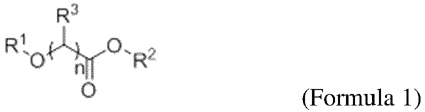 wherein R¹ and R² are each independently one of an alkyl group having 1 to 4 carbon atoms or an aryl group, wherein R³ is hydrogen, methyl, or ethyl, and wherein n is 1 to 6. In some embodiments, R¹ and R² are ethyl groups, R³ is hydrogen, and n is 2.

The present invention relates to pesticide formulations. The pesticide formulation comprises a pesticide and a solvent according to any of the embodiments disclosed herein. In some embodiments, the solvent comprises 1 to 90 weight percent of the formulation and the pesticide comprises 10 to 99 weight percent of the formulation. The solvent, in some embodiments, comprises 2 to 70 weight percent of the formulation, and the pesticide comprises 30 to 98 weight percent of the formulation. In some embodiments, the pesticide comprises one or more of chlopyrifos, tebuconazole, difenoconazole, triazolone, quizalofop-p-ethyl, myclobutanil, pyraclostrobin, bifenthrin, buprofezin, and beta-cypermethrin.

### Solvents for Pesticides

Solvents for pesticides according to some embodiments described herein comprise an ether ester of Formula 1: 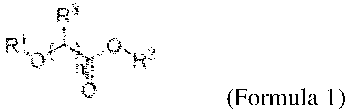 wherein R¹ and R² are each independently one of an alkyl group having 1 to 4 carbon atoms or an aryl group, wherein R³ is hydrogen, methyl, or ethyl, and wherein n is 1 to 6. In some embodiments, R¹ and R² are ethyl groups, R³ is hydrogen, and n is 2.

Solvents for pesticides according to some embodiments described herein consist of an ether ester of Formula 1: 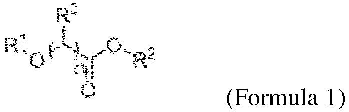 wherein R¹ and R² are each independently one of an alkyl group having 1 to 4 carbon atoms or an aryl group, wherein R³ is hydrogen, methyl, or ethyl, and wherein n is 1 to 6. In some embodiments, R¹ and R² are ethyl groups, R³ is hydrogen, and n is 2.

Solvents for pesticides according to some embodiments described herein consist essentially of an ether ester of Formula 1: 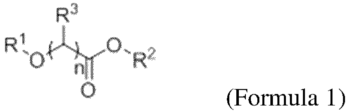 wherein R¹ and R² are each independently one of an alkyl group having 1 to 4 carbon atoms or an aryl group, wherein R³ is hydrogen, methyl, or ethyl, and wherein n is 1 to 6. In some embodiments, R¹ and R² are ethyl groups, R³ is hydrogen, and n is 2.

In one embodiment the ether ester is ethyl-3-ethoxy propionate and are commercially available from The Dow Chemical Company as UCAR^{™} Ester EEP.

Solvents for pesticides described herein can comprise, consist of, or consist essentially of two or more ether esters of Formula 1. "Two or more ether esters of Formula 1" means that the solvent comprises at least two distinct ether esters of Formula 1 (e.g., a first ether ester in which R¹ and R² methyl and a second ether ester in which R¹ and R² are ethyl; or a first ether ester in which n is 2 and a second ether ester in which n is 3 and all other substituents or components of the two ether esters are the same; etc. The ether esters can differ from one another in more than one substituent or component, and the solvent can comprise any number of different ether esters of Formula 1.

In some embodiments, solvents described herein comprise or consist essentially of at least 95 percent by weight of ether esters of Formula 1. Solvents of the present invention, in other embodiments, comprise or consist essentially of at least 95 weight percent, or at least 97 weight percent, or at least 98 weight percent or at least 99 weight percent, or at least 99.9 weight percent of ether esters of Formula 1.

In some embodiments, solvents described herein comprise or consist essentially of at least 95 percent by weight ethyl-3-ethoxy propionate. Solvents of the present invention, in other embodiments, comprise or consist essentially of at least 95 weight percent, or at least 97 weight percent, or at least 98 weight percent or at least 99 weight percent, or at least 99.9 weight percent of ethyl-3-ethoxy propionate.

### Pesticide Formulations

Pesticide formulations according to the present invention comprise a pesticide and a solvent according to any of the embodiments of solvents for pesticides disclosed herein. The pesticides comprises one or more of , chlopyrifos, tebuconazole, difenoconazole, triazolone, quizalofop-p-ethyl, myclobutanil, pyraclostrobin, bifenthrin, buprofezin, and beta-cypermethrin.

In some embodiments, a pesticide formulation comprises 1 to 90 weight of a solvent according to any of the solvents for pesticides disclosed herein and 10 to 99 weight percent of the pesticide, each based on the total weight of the formulation. In some embodiments, a pesticide formulation comprises 2 to 70 weight of the solvent and 30 to 98 weight percent of the pesticide, each based on the total weight of the formulation.

In some embodiments, a pesticide formulation consists of 1 to 90 weight of a solvent according to any of the solvents for pesticides disclosed herein and 10 to 99 weight percent of the pesticide, each based on the total weight of the formulation. In some embodiments, a pesticide formulation consists of 2 to 70 weight of the solvent and 30 to 98 weight percent of the pesticide, each based on the total weight of the formulation.

In some embodiments, a pesticide formulation consists essentially of 1 to 90 weight of a solvent according to any of the solvents for pesticides disclosed herein and 10 to 99 weight percent of the pesticide, each based on the total weight of the formulation. In some embodiments, a pesticide formulation consists essentially of 2 to 70 weight of the solvent and 30 to 98 weight percent of the pesticide, each based on the total weight of the formulation.

Optional materials that are not essential to the operability of, but can be included in, the pesticide formulations of this invention includeantioxidants, colorants, water scavengers, stabilizers.. These materials do not have any material impact on the efficacy of the pesticide formulation. These optional materials are used in known amounts, e.g., 0.10 to 5, or 4, or 3, or 2, or 1, weight percent based on the weight of the solvent, and they are used in known ways.

Pesticide formulations can be prepared using techniques known to those of skill in the art. For example, the active ingredient (pesticide) can be dissolved in a solvent, and then emulsifiers or other conventional additives can be added.

Some embodiments of the invention will now be described in detail in the following Examples.

### Examples

The following examples are given to illustrate the invention and should not be construed as limiting its scope. All parts and percentages are by weight unless otherwise indicated.

The following solvents in Table 1 are evaluated for performance as a solvent for pesticides:

**Table 1**

| **Solvent** | **Chemical** |
|---|---|
| Comparative Solvent A | xylene |
| Comparative Solvent B | Solvesso 150 |
| Inventive Solvent 1 | ethyl 3-ethoxypropionate |

The xylene is from Sinopharm, Co., China. Solvesso 150 is an aromatic solvent (CAS No. 64742-94-5) from ExxonMobil Chemical Co. The ethyl 3-ethoxypropionate is UCAR^{™} Ester EEP available from The Dow Chemical Company. The solubility of various pesticides in these solvents are evaluated. The pesticides are shown in Table 2:

**Table 2**

| **Components** | **Supplier** |
|---|---|
| Chlopyrifos | Zhongye Chemical Co. (China) |
| Tebuconazole | Liannong Chemical Co. (China) |
| Difenoconazole | Suli Chemical Co. (China) |
| Triazolone | Qizhou Chemical Co. (China) |
| Quizalofop-p-ethyl | Jingbo Chemical Co. (China) |
| Myclobutanil | Shengya Chemical Co. (China) |
| Pyraclostrobin | Shentai Chemical Co. (China) |
| Bifenthrin | Lier Chemical Co. (China) |
| Buprofezin | Anbang Chemical Co. (China) |
| Beta-cypermethrin | Tianyuan Bio-chemical Co. (China) |

Each pesticide powder is added by solvent in 2% stepwise increments to the final pesticide concentration. Each pesticide reaches a concentration where the pesticide powder is not dissolved by the solvent. The concentration just prior to the concentration where the pesticide powder is not dissolved is shown in Table 3. Thus, the data in Table 3 reflects a solubility that is no more than 2% lower than the actual solubility.

**Table 3**

| **Active Ingredients** | **Comparative Solvent A (weight %)** | **Comparative Solvent B (weight %)** | **Inventive Solvent 1 (weight %)** |
|---|---|---|---|
| Chlopyrifos | 70 | 54 | 63 |
| Tebuconazole | 4.0 | 2.0 | 16 |
| Difenoconazole | 34 | 19 | 33 |
| Triazolone | 23 | 12 | 32 |
| Quizalofop-p-ethyl | 32 | 23 | 22 |
| Myclobutanil | 27 | 23 | 39 |
| Pyraclostrobin | 42 | 31 | 47 |
| Bifenthrin | 45 | 44 | 43 |
| Buprofezin | 10 | 16 | 18 |
| Beta-cypermethrin | 32 | 34 | 43 |

In comparing the dissolving ability of Comparative Solvent A and Inventive Solvent 1, Comparative Solvent A has a stronger dissolving ability than Inventive Solvent 1 for some active ingredients (e.g., chlopyrifos, quizalofop-p-ethyl and bifenthrin). However, for the rest of evaluated active ingredients, Inventive Solvent 1 exhibits better solubility than Comparative Solvent A. Overall, Inventive Solvent 1 is believed to exhibit comparable dissolving performance relative to Comparative Solvent A with respect to the various pesticides evaluated. While exhibiting comparable dissolving performance, Inventive Solvent 1 is also advantageously believed to be more environmentally friendly than Comparative Solvent A.

In comparing the dissolving ability of Comparative Solvent B and Inventive Solvent 1, the two solvents have comparable dissolving ability as to three of the evaluated active ingredients (buprofezin, bifenthrin and quizalofop-p-ethyl). For the other active ingredients, Inventive Solvent 1 provides much better solubility performance than Comparative Solvent B. Thus, Inventive Solvent 1 has a stronger dissolving ability for these active ingredients than Comparative Solvent B. While exhibiting generally stronger dissolving performance, Inventive Solvent 1 is also advantageously believed to be more environmentally friendly than Comparative Solvent B.

## Claims

1. A pesticide formulation comprising:
a solvent comprising an ether ester of Formula 1: 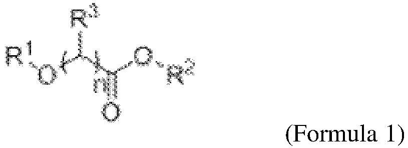 wherein R¹ and R² are each independently one of an alkyl group having 1 to 4 carbon atoms or an aryl group, wherein R³ is hydrogen, methyl, or ethyl, and wherein n is 1 to 6; and
a pesticide; wherein the pesticide comprises one or more of chlopyrifos, tebuconazole, difenoconazole, triazolone, quizalofop-p-ethyl, myclobutanil, pyraclostrobin, bifenthrin, buprofezin, and beta-cypermethrin.

2. The pesticide formulation of claim 1, wherein the solvent comprises 1 to 90 weight percent of the formulation and wherein the pesticide comprises 10 to 99 weight percent of the formulation.

3. The pesticide formulation of claim 1 or 2, wherein in the solvent R¹ and R² are ethyl groups, R³ is hydrogen, and wherein n is 2.

4. The pesticide formulation of any one of claims 1 to 3, wherein the solvent comprises two or more ether esters of Formula 1.

## Patentansprüche

1. Pestizidformulierung, umfassend:
ein Lösungsmittel, umfassend einen Etherester der Formel 1: 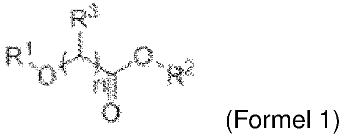
wobei R¹ und R² jeweils unabhängig eine von einer Alkylgruppe, die 1 bis 4 Kohlenstoffatome aufweist, oder einer Arylgruppe sind, wobei R³ Wasserstoff, Methyl oder Ethyl ist und wobei n 1 bis 6 ist; und
ein Pestizid; wobei das Pestizid eines oder mehrere von Chlopyrifos, Tebuconazol, Difenoconazol, Triazolon, Quizalofop-p-ethyl, Myclobutanil, Pyraclostrobin, Bifenthrin, Buprofezin, und beta-Cypermethrin umfasst.

2. Pestizidformulierung nach Anspruch 1, wobei das Lösungsmittel zu 1 bis 90 Gewichtsprozent die Formulierung umfasst und wobei das Pestizid zu 10 bis 99 Gewichtsprozent die Formulierung umfasst.

3. Pestizidformulierung nach Anspruch 1 oder 2, wobei in dem Lösungmsittel R¹ und R² Ethylgruppen sind, R³ Wasserstoff ist, und wobei n 2 ist.

4. Pestizidformulierung nach einem der Ansprüche 1 bis 3, wobei das Lösungsmittel zwei oder mehr Etherester der Formel 1 umfasst.

## Revendications

1. Formulation de pesticide comprenant :
un solvant comprenant un ester d'éther de Formule 1 : 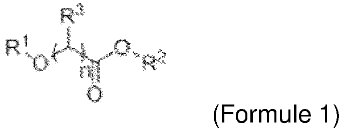
dans laquelle R¹ et R² sont chacun indépendamment l'un parmi un groupe alkyle ayant 1 à 4 atomes de carbone ou un groupe aryle, dans laquelle R³ est hydrogène, méthyle, ou éthyle, et dans laquelle n vaut de 1 à 6 ; et
un pesticide ; dans laquelle le pesticide comprend un ou plusieurs parmi chlopyrifos, tébuconazole, difénoconazole, triazolone, quizalofop-p-éthyle, myclobutanil, pyraclostrobine, bifenthrine, buprofézine, et bêta-cyperméthrine.

2. Formulation de pesticide selon la revendication 1, dans laquelle le solvant représente de 1 à 90 pour cent en poids de la formulation et dans laquelle le pesticide représente de 10 à 99 pour cent en poids de la formulation.

3. Formulation de pesticide selon la revendication 1 ou 2, dans laquelle, dans le solvant, R¹ et R² sont des groupes éthyle, R³ est hydrogène, et dans laquelle n vaut 2.

4. Formulation de pesticide selon l'une quelconque des revendications 1 à 3, dans laquelle le solvant comprend deux esters d'éther de la Formule 1 ou plus.
